# EUROPEAN PATENT SPECIFICATION

(11) **EP 1 177 157 B1**
(45) Date of publication and mention of the grant of the patent: **23.05.2007**
(21) Application number: 00908096.1
(22) Date of filing: 07.03.2000
(51) Int. Cl.: C04B 33/04, C04B 22/14, A01G 31/00, C08K 3/00, C08K 3/34

(54) **A VITAL MATTER AND A PRODUCING METHOD**
ESSENTIELLES MATERIAL UND VERFAHREN ZU DESSEN HERSTELLUNG
MATIERE ESSENTIELLE ET PROCEDE DE PRODUCTION ASSOCIE

(30) Priority: 09.03.1999 KR 9907707
(43) Date of publication of application: 06.02.2002
(73) Proprietor: Song, Si-Hoon, Chollabuk-dp 570-380 (KR)
(72) Inventor: Song, Si-Hoon, Chollabuk-dp 570-380 (KR)
(74) Representative: Andrews, Timothy Stephen
(86) International application number: PCT/KR2000/000177
(87) International publication number: WO 2000/053541

(56) References cited:
- EP-A- 0 893 475
- WO-A-98/14050
- GB-A- 631 819
- JP-A- 62 182 163
- KR-A- 9 614 048
- US-A- 4 960 737
- US-A- 5 328 498

## Description

### FIELD OF THE INVENTION

The present invention relates to a vital matter for human body, animals and plants promoting their growth and increasing preservative capability of animals and plants.

The present invention also relates to a producing method of the vital matter composed of natural substances and compounds by mixing at almost the same ratio as that of inorganic substances in human, animals and plants.

The producing method of the present invention may be used in the whole field of industries such as building materials, things of life, a medical industry and a food industry.

### BACKGROUND

Natural substances such as yellow soil and silicon dioxide mineral, and synthetic ceramic have been used in the whole field of industries such as medical instruments using infrared rays and things of life.

However, since the above-mentioned things is prepared by using the natural substances such as yellow soil and white soil as major components, content of a silicate (SiO₂) is high, whereas contents of inorganic substances such as potasium, calcium, sodium, magnesium and iron are very low. Thus, it is impossible to accomplish sympathy of energy and native wavelength between conventional substances and human body, animals and plants.

### SUMMARY OF THE INVENTION

It is an object of this invention to provide a vital matter activating original active rhythm of human body, animals and plants at a maximum level.

It is a further object of this invention to provide a producing method the vital matter.

### DETAILED DESCRIPTION OF PREFERRED EMBODIMENTS

Since the vital matter of the present invention as defined in the claims 1-5 has a similar composition to a major inorganic substance of human body, animals and plants, the vital matter induces a resonance phenomenon by approaching to human body, animals and plants, so that sympathy of energy and native wavelength between it and human body, animals and plants is maximized.

In detail, when five or six bronze bells made from the same materials are hang and one of them rings, others ring with the same sound, which is a resonance phenomenon. The resonance phenomenon also occurs when drums or bowls made from the same materials are used for the above experiment. However, the resonance phenomenon does not occur if a drum or a bowl rings and vice versa. Therefore, it is demonstrated that things made from the same materials induce sympathy of energy and native wavelength.

Otherwise, potassium, calcium, sodium, magnesium and iron are major components of inorganic substances of human body, animals and plants. Thus, the composition of the present invention is prepared by mixing various components at almost the same ratio as that of inorganic components of human body, animals and plants. Sympathy of energy and native wavelength between the composition of the present invention and human body, animals and plants, is maximized to activate active rhythm of human body, animals and plants at maximal level.

The composition of the present invention contains kaoline(white soil) 30.0-40.0wt%, potassium sulfate 15.0-20.0wt%, sodium sulfate 13.0-17.0wt%, feldspar 12.0-16.0wt%, talc 12.0-16.0% and ferric oxide 0.5-1.5wt%. The composition is mixed by a compressed molding method with water, dried and manufactured in random forms. The resulting composition becomes plastic at 1000-1300°C for its use in various forms.

The vital matter of the present invention prepared by the above-mentioned composition has components shown in the following Table 1.

**<Table 1> Average ratio of components of composition**

| **Components** | **Weight ratio** (wt%) |
|---|---|
| Potassium(K) | 19.06-23.29wt% |
| Calcium(Ca) | 14.21-17.36wt% |
| Sodium(Na) | 12.30-14.97wt% |
| Magnesium(Mg) | 11.98-14.64wt% |
| Silicon(Si) | 13.74-16.80wt% |
| Aluminum (Al) | 12.21-15.13wt% |
| Iron(Fe) | 3.48-4.26wt% |
| Titanium(Ti) | 0.95-1.17wt% |
| Manganese(Mn) | 0.28-0.40wt% |
| Zinc(Zn) | 0.17-0.20wt% |
| Germanium(Ge) | 0.07-0.09wt% |
| Selenium(Se) | 0.03-0.04wt% |
| Other elements | 1.36-1.67wt% |

The major components of the composition of the present invention are potassium, calcium, sodium and magnesium, which is similar distribution with inorganic substances of human body, animals and plants. In addition, the composition of the present invention has an affinity for silicon and aluminium abundantly contained in soil.

Whereas, as shown in Table 2, general ceramic products contain large amounts of silicon and aluminium, and small amounts of potassium, calcium, sodium and magnesium.

**<Table 2> Average ratio of components of general ceramic products**

| **Components** | **Weight ratio (wt%)** |
|---|---|
| Aluminium(A1) | 35.36-43.22wt% |
| Silicon(Si) | 31.33-38.30wt% |
| Potassium(K) | 7.73-9.45wt% |
| Magnesium(Mg) | 3.56-4.36wt% |
| Iron(Fe) | 3.52-4.31wt% |
| Calcium(Ca) | 3.40-4.16wt% |
| Sodium(Na) | 2.79-3.63wt% |
| Titanium(Ti) | 0.03-0.04wt% |
| Other elements | 2.10-2.57wt% |

The ratio of components of general yellow soil ceramic is shown in Table 3.

**<Table 3> Average ratio of components of general yellow soil ceramic**

| **Components** | **Weight ratio (wt%)** |
|---|---|
| Silicon dioxide (SiO₂) | 64.08-79.42wt% |
| Aluminium oxide ((Al₂O₃) | 9.45-11.55wt% |
| Sodium oxide(NaO₂) | 3.32-4.02wt% |
| Ferric oxide ( Fe₂O₃) | 2.93-3.58wt% |
| Potassium oxide (K₂O) | 2.22-2.71wt% |
| Other elements | 8.02-9.80wt% |

As shown in Table 2 and 3, the general ceramic and the general yellow soil ceramic contains mostly silicon and aluminium as major components, and small amounts of potassium, calcium, sodium and magnesium which are associated with human body, animals and plants. Thus, Sympathy of energy and native wavelength between the general ceramic or the general yellow soil ceramic and human body, animals and plants, does not occur.

Hereinafter, the present invention is described in detail.

### EXAMPLES

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Preparation of the vital matter

The composition of the present invention contains the following components: i) Kaoline (white soil) 30-40wt%; ii) potassium sulfate 15.0-20.0wt%; iii) sodium sulfate 13.0-17.0wt%; iv) feldspar 12.0-16.0wt%; v) talc 12.0-16.0%; and vi) ferric oxide 0.5-1.5wt%.

In the above composition, potassium sulfate and sodium sulfate may be replaced by the same amounts of potassium chloride and sodium chloride ions. However, because a moisture drying efficiency of sulfate salts are better than that of chloride salts, the present inventors selected potassium sulfate and sodium sulfate to increase the moisture drying efficiency.

The composition was manufactured in form of minute powder of 100-150 mesh. After the composition was mixed by the compressed molding method or with 20-30wt% of water to mold in the fixed form, it was dried by hot wind at 40-80°C for 10-15 hours and heated 1000-1300°C for 2-3 hours to be plastic.

The manufactured composition was prepared in various form to be used for various industry.

The composition of the present invention activated active rhythm of human body, animals and plants at a maximum level by inducing sympathy of energy and native wavelength between it and human body, animals and plants. In addition, this activation by the composition of the present invention was superior to that by conventional ceramic products.

Generally, infrared rays irradiation of silicon is higher than that of potassium. Whereas, the composition of the present invention was excellent in bioaffinity and sympathy of energy and native wavelength between it and human body, animals and plants.

### Experiment 1: Physiological reactivity of the composition of the present invention and general ceramic products

The present inventors performed the physiological reactivity experiment of the composition and general ceramic products, and compared their physiological reactivities. The result was shown in Table 4.

**<Table 4> The results of comparing the physiological reactivity.**

| Item | Refinement velocity of coffee taste | Refinement velocity of tobacco | Deordorization of Refrigerator | Freshness of vegetables |
|---|---|---|---|---|
| Yellow soil ceramic | 10 hours* (3 hours) | 10 hours* (3 hours) | No effect | No effect |
| Medical ceramic | 10 min* (20 sec) | 5 min* (5 sec) | From 2 hours after starting | 180% increase |
| Industrial ceramic | 5 hours* (1 hour) | 1 hour* (30 min) | From 5 hours after starting | 130% increase |
| The composition of the present invention | 30 sec* (10 sec) | 20 sec* (2 sec) | From 30 min after starting | 250% increase |

| | | | | |
|---|---|---|---|---|
| <*:the experiment was performed at room temperature, ( ): the experiment was performed at 50°C | | | | |

The composition of the present invention was superior to the conventional ceramic products in acting velocity and efficiency of refinement toward adventages of living body.

In addition, the composition was prepared in form of minute powder of 200-350 mesh and mixed with synthetic resin to the concentration of 5-30%. The resulting mixture can be used in various forms for industry:

For example, after the composition of the present invention was added to polyethylene film which has been used a vinyl house for cultivating plants, the present inventors cultivated the crops using the vinyl house made from the ployethylene film containing the composition of the present invention and the vinyl house made from general polyethylene film. The results was shown in Table 5.

**<Table 5> The results of cultivating the crops**

| crop | Average yield | | |
|---|---|---|---|
| | Polyethylene film | Polyethylene film containing the component | Comparison (increasing ratio) |
| Chinese cabbage | 416 kg | 499 kg | 20% increase |
| Cucumber | 422 kg | 527 kg | 25% increase |
| Tomato | 575 kg | 719 kg | 25% increase |
| Red pepper | 179 kg | 250 kg | 40% increase |

| | | | |
|---|---|---|---|
| (increase per 100 m² of cultivation areas) | | | |

As shown in Table 5, when the synthetic resin containing the compositin of the present invention was used, the yield of the crops was increased more about 20-40% than that when the general synthetic resin was used. Therefore, these results demonstrate that, the composition of the present invention accelerates physiological activity of plants.

### INDUSTRIAL APPLICABILITY

The composition of the present invention, a vital matter for human body, animals and plants, can maximize sympathy of an activation energy and a native wavelength between it and human body, animals and plants. Thus, the composition of the present invention can be used for industry and will cause the original changes in the field of industrial matters.

In detail, for example, the composition of the present invention can be used all the industries including building materials and raw materials of various synthetic resins (especially, vinyl, plastic, etc.), various food containers, cosmetics and cosmetics containers, various medical instruments (especially, medical instruments using far infrared rays), medicines and medicines containers, containers for cultivating various plants, deordorants and chemical products such as agricultural chemicals. Therefore, it is expected that the composition of the present invention, the vital matter for human body, animals and plants, will promote the welfare of human beings such as improvement of health and life of human.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

## Claims

1. A vital matter obtainable by mixing kaoline (white soil) 30.0-40.0 wt%, potassium sulfate 15.0-20.0 wt%, sodium sulfate 13.0-17.0 wt%, feldspar 12.0-16.0 wt%, talc 12.0-1,6.0 wt% and ferric oxide 0.5-1.5 wt% using a compressed molding method and then heating the resulting mixture at 1000-1300°C.

2. A vital matter obtainable by mixing kaoline (white soil) 30.0-40.0 wt%, potassium chloride 15.0-20.0 wt%, sodium chloride 13.0-17.0 wt%, feldspar 12.0-16.0 wt%, talc 12.0-16.0 wt% and ferric oxide 0.5-1.5 wt% using a compressed molding method and then heating the resulting mixture at 1000-1300°C.

3. A vital matter according to claim 1 or claim 2 comprising potassium 19.06-23.29 wt%, calcium 14.21-17.36 wt%, sodium 12.30-24.97 wt%, magnesium 11.98-14.64 wt%, silicon 13.74-16.80 wt%, aluminum 12.21-15.13 wt%, iron 3.48-4.26 wt%, titanium 0.95-1.17 wt%, manganese 0.28-0.40 wt%, zinc 0.17-0.20 wt%, germanium 0.07-0.09 wt%, selenium 0.03-0.04 wt% and other elements 1.36-1.67 wt%.

4. A vital matter according to any one of claim 1 to 3, wherein said vital matter is prepared in the form of minute powder of 200-350 mesh and mixed with a synthetic resin.

5. A vital matter according to claim 4, wherein the concentration of said resin is 5 to 30%.

6. A polyethylene film containing the vital, matter according to any one of claims 1 to 3.

7. A method of preparing a vital matter, comprising the steps: 1) mixing kaoline (white soil) 30.0-40.0 wt%, potassium sulfate 15.0-20.0 wt%, sodium sulfate 13.0-17.0 wt%, feldspar 12.0-16.0 wt%, talc 12.0-16.0 wt% and ferric oxide 0.5-1.5 wt% using a compressed molding method; and 2) heating the mixture at 1000-1300°C.

8. A method of preparing a vital matter according to claim 7, wherein said heating is carried out for a time period of 2 to 3 hours.

## Patentansprüche

1. Vitalstoff, der erhalten werden kann durch Mischen von 30% bis 40 Gew.% Kaolin (weißem Ton), 15% bis 20 Gew.% Kaliumsulfat, 13% bis 17 Gew.% Natriumsulfat, 12% bis 16 Gew.% Feldspat, 12% bis 16 Gew.% Talkum und 0,5% bis 1,5 Gew.% Eisen(III)-oxid und Anwendung eines Verfahrens des Formpressens und anschließendem Erhitzen der resultierenden Mischung bei 1.000° bis 1.300°C.

2. Vitalstoff, der erhalten werden kann durch Mischen von 30% bis 40 Gew.% Kaolin (weißem Ton), 15% bis 20 Gew.% Kaliumchlorid, 13% bis 17 Gew.% Natriumchlorid, 12% bis 16 Gew.% Feldspat, 12% bis 16 Gew.% Talkum und 0,5% bis 1,5 Gew.% Eisen(III)-oxid und Anwendung eines Verfahrens des Formpressens und anschließendem Erhitzen der resultierenden Mischung bei 1.000° bis 1.300°C.

3. Vitalstoff nach Anspruch 1 oder 2, aufweisend: 19,06% bis 23,29 Gew.% Kalium, 14,21% bis 17,36 Gew.% Calcium, 12,30% bis 14,97 Gew.% Natrium, 11,98% bis 14,64 Gew.% Magnesium, 13,74% bis 16,80 Gew.% Silicium, 12,21% bis 15,13 Gew.% Aluminium, 3,48% bis 4,26 Gew.% Eisen, 0,95% bis 1,17 Gew.% Titan, 0,28% bis 0,40 Gew.% Mangan, 0,17% bis 0,20 Gew.% Zink, 0,07% bis 0,09 Gew.% Germanium, 0,03% bis 0,04 Gew.% Selen und 1,36% bis 1,67 Gew.% andere Elemente.

4. Vitalstoff nach einem der Ansprüche 1 bis 3, wobei der Vitalstoff in Form eines feinteiligen Pulvers von 200 bis 350 Mesh hergestellt und mit einem synthetischen Harz gemischt wird.

5. Vitalstoff nach Anspruch 4, worin die Konzentration des Harzes 5% bis 30 Gew.% beträgt.

6. Polyethylen-Folie, enthaltend den Vitalstoff nach einem der Ansprüche 1 bis 3.

7. Verfahren zum Herstellen eines Vitalstoffes, umfassend die Schritte: 1) Mischen von 30% bis 40 Gew.% Kaolin (weißem Ton), 15% bis 20 Gew.% Kaliumsulfat, 13% bis 17 Gew.% Natriumsulfat, 12% bis 16 Gew.% Feldspat, 12% bis 16 Gew.% Talkum und 0,5% bis 1,5 Gew.% Eisen(III)-oxid und Anwendung eines Verfahrens des Formpressens und 2) Erhitzen der resultierenden Mischung bei 1.000° bis 1.300°C.

8. Verfahren zum Herstellen eines Vitalstoffes nach Anspruch 7, wobei das Erhitzen für eine Zeitdauer von 2 bis 3 Stunden ausgeführt wird.

## Revendications

1. Matière essentielle qui peut être obtenue en mélangeant de 30,0 à 40,0% en poids de kaolin (terre blanche), de 15,0 à 20,0% en poids de sulfate de potassium, de 13,0 à 17,0% en poids de sulfate de sodium, de 12,0 à 16,0% en poids de feldspath, de 12,0 à 16,0% en poids de talc et de 0,5 à 1,5% en poids d'oxyde ferrique en utilisant une méthode de moulage par compression et en faisant ensuite chauffer le mélange résultant à 1 000-1 300°C.

2. Matière essentielle qui peut être obtenue en mélangeant de 30,0 à 40,0% en poids de kaolin (terre blanche), de 15,0 à 20,0% en poids de chlorure de potassium, de 13,0 à 17,0% en poids de chlorure de sodium, de 12,0 à 16,0% en poids de feldspath, de 12,0 à 16,0% en poids de talc et de 0,5 à 1,5% en poids d'oxyde ferrique en utilisant une méthode de moulage par compression et en faisant ensuite chauffer le mélange résultant à 1 000-1 300°C.

3. Matière essentielle selon la revendication 1 ou la revendication 2 qui comprend de 19,06 à 23,29% en poids de potassium, de 14,21 à 17,36% en poids de calcium, de 12,30 à 14,97% en poids de sodium, de 11,98 à 14,64% en poids de magnésium, de 13,74 à 16,80% en poids de silicium, de 12,21 à 15,13% en poids d'aluminium, de 3,48 à 4,26% en poids de fer, de 0,95 à 1,17% en poids de titane, de 0,28 à 0,40% en poids de manganèse, de 0,17 à 0,20% en poids de zinc, de 0,07 à 0,09% en poids de germanium, de 0,03 à 0,04% en poids de sélénium et de 1,36 à 1,67% en poids d'autres éléments.

4. Matière essentielle selon l'une quelconque des revendications 1 à 3, où ladite matière essentielle est préparée sous la forme d'une poudre extrêmement fine de 200-350 meshs et mélangée à une résine synthétique.

5. Matière essentielle selon la revendication 4, où la concentration de ladite résine est comprise entre 5 et 30%.

6. Film polyéthylène contenant la matière essentielle selon l'une quelconque des revendications 1 à 3.

7. Procédé de préparation d'une matière essentielle qui comprend les étapes consistant à : 1) mélanger de 30,0 à 40,0% en poids de kaolin (terre blanche), de 15,0 à 20,0% en poids de sulfate de potassium, de 13,0 à 17,0% en poids de sulfate de sodium, de 12,0 à 16,0% en poids de feldspath, de 12,0 à 16,0% en poids de talc et de 0,5 à 1,5% en poids d'oxyde ferrique en utilisant une méthode de moulage par compression et 2) en faisant chauffer le mélange à 1 000-1 300°C.

8. Procédé de préparation d'une matière essentielle selon la revendication 7, où ladite chauffe est effectuée sur une période de temps de 2 à 3 heures.
